# EUROPEAN PATENT SPECIFICATION

(11) **EP 0 597 429 B1**
(45) Date of publication and mention of the grant of the patent: **26.02.2003**
(21) Application number: 93118094.7
(22) Date of filing: 08.11.1993
(51) Int. Cl.: C07D 501/18, C07D 501/22, C07D 501/24

(54) **Process for the production of 3-vinylcephalosporins**
Verfahren zur Herstellung von 3-Vinylcephalosporinen
Procédé pour la préparation de 3-vinylcéphalosporines

(30) Priority: 10.11.1992 AT 221292
(43) Date of publication of application: 18.05.1994
(73) Proprietor: BIOCHEMIE GESELLSCHAFT M.B.H., 6250 Kundl (AT)
(72) Inventor: Wieser, Josef, A-6330 Kufstein (AT)
(74) Representative: Schaller, Hans

(56) References cited:
- EP-A- 0 503 453
- MARCH J.: 'Advanced Organic Chemistry, 2nd edition', MCGRAW-HILL INTERNATIONAL BOOK CO., AUCKLAND ET AL.
- BUEHLER C.A. ET AL.: 'Survey of Organic Syntheses', WILEY-INTERSCIENCE, NEW YORK ET AL.

## Description

The invention relates to an economical and simple process for the production of 3-vinylcephalosporin compounds of formula I 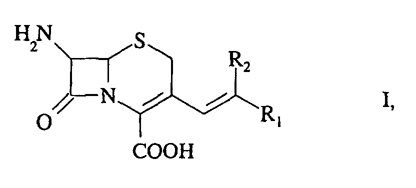 wherein R₁ and R₂ may be the same or different and denote hydrogen or an optionally branched alkyl, alkenyl or alkinyl group, each containing up to 10 carbon atoms; a totally or partially saturated cycloalkyl radical containing up to 10 carbon atoms; or an optionally substituted aryl radical, aralkyl radical or heterocycle, each containing up to 10 carbon atoms. The cycloalkyl radical, aryl radical, aralkyl radical or heterocycle each containing up to 10 carbon atoms may be substituted in any position, for example by halogen, nitrogen, sulphur, alkoxy, aryloxy, or a functional group such as a carbalkoxy or carboxamido group.

The compounds of formula I are known to be useful starting products for the production of valuable 3-substituted vinyl cephalosporins.

In a preferred embodiment of the invention one of R₁ and R₂ is
hydrogen and the other is:
i) hydrogen, lower alkyl, lower alkenyl, or lower alkinyl;
ii) lower cycloalkyl, lower cycloalkyl lower alkyl, aryl, (aryl)-lower alkyl, a heterocyclic group or a heterocyclyl-(lower)-alkyl, the ring of each of which may be optionally substituted by 1 to 3 lower alkoxy, lower alkylthio, halogen, lower alkyl, nitro, hydroxy, acyloxy, carboxy, carbalkoxy, lower alkylcarbonyl, lower alkylsulfonyl, lower alkoxysulfonyl, amino-(lower)-alkyl amino or acylamido groups; or
iii) a group of formula -CH₂Z, in which Z is a) hydroxy, lower alkoxy, formyloxy, acetyloxy, lower alkylsulfonyloxy, halogen, N-mono(lower)alkylcarbamoyloxy, or N,N-di(lower)alkylcarbamoyloxy; b) a heterocyclic group; c) a group of formula -S(O)ₘR₉ in which R₉ is an aliphatic, araliphatic, alicyclic, aromatic or heterocyclic group, and m is 0, 1 or 2; or d) an acyclic or cyclic ammonium group.

Suitable heterocyclic groups include single or fused heterocyclic rings having 4 to 7, preferably 5- or 6- atoms in each ring. Each ring has up to four hetero atoms in it selected from oxygen, nitrogen and sulphur. Also each heterocyclic ring may have 1 to 3 optional substituents selected from (C₁₋₄) alkyl, (C₁₋₄) alkoxy, halogen, trihalo-(C₁₋₄) alkyl, hydroxy, oxo, mercapto, amino, carboxyl, carbamoyl, di-(C₁₋₄) alkylamino, carboxymethyl, carbamoylmethyl, sulfomethyl and methoxycarbonylamino.

Examples of suitable heterocycle rings include unsubstituted and substituted imidazolyl, diazolyl, triazolyl, tetrazolyl, thiazolyl, thiadiazolyl, thiatriazolyl, oxazolyl, oxadiazolyl, benzimidazolyl, benzoxazolyl, benzothiazolyl, triazolylpyridyl, purinyl, pyridyl, pyrimidinyl, pyridazinyl, pyrazolyl and triazinyl.

Preferably, suitable heterocycle rings include unsubstituted and substituted 5-hydroxy-4-pyridon-2-yl, 1,2,3-triazolyl; 1,2,4-triazolyl; tetrazolyl; oxazolyl; thiazolyl; 1,3,4-oxadiazolyl; 1,3,4-thiadiazolyl or 1,2,3-thiadiazolyl.Preferably the heterocycle is 1,5-dihydroxy-4-pyridon-2-yl, 5-hydroxy-1-methyl-4-pyridon-2-yl, 5-hydroxy-4-pyridon-2-yl, 1-methyl-1H-tetrazol-5-yl-2-methyl-1,3,4-thiadiazol-5-yl, 1-carboxymethyl-1H-tetrazol-5-yl, 6-hydroxy-2-methyl-5-oxo-2H-1,2,4-triazin-3-yl, 1,2,3-triazol-5-yl, and 4-methyl-thiazol-5-yl.

Examples of acyclic ammonium groups include (1-carbamoyl-2--hydroxyethyl)-dimethylammonium, (carbamoylmethyl)(ethyl)-methylammonium or trimethyl ammonium.

Examples of cyclic ammonium groups are pyrrolidinium, which is N-substituted by alkyl, carbamoylalkyl, aminoalkyl or carboxyalkyl; pyridinium or cyclopentenopyridinium, which may be mono- or di-substituted by alkyl, halogen, hydroxy, carboxamido, alkoxycarbonyl, amino, monoalkylamino or dialkylamino.

Except where otherwise indicated, the organic radicals contain up to 10 carbon atoms and "lower" means the group has up to 4 carbon atoms.

Processes for the production of compounds of formula I are known and are discussed in EP 0503453, the disclosure of which is incorporated by reference. However, as discussed in EP 0503453, these known processes require the use of expensive protection groups and require a multiplicity of intermediate stages. The invention disclosed in EP 0503453 addressed the problems of the prior art by making use of silyl protection groups in a Wittig reaction using 7-amino cephalosporanic acid as starting reagent.

The process disclosed in EP 0503453 proceeds according to the following reaction scheme:
i) a compound of the formula II 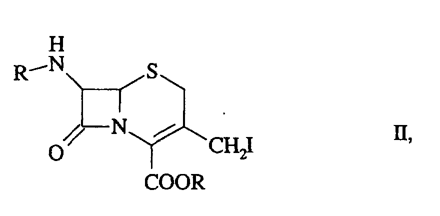 in which R is a silyl protecting group, is reacted with a compound of the formula P(R₄)₃ or P(OR₄)₃ to produce a compound of formula 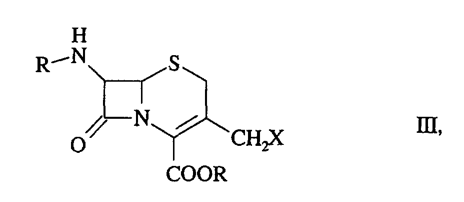 in which X is -P(R₄)₃I or -P(O)-(OR₄)₂, R is as defined above and R₄ is a lower alkyl group or an aryl group;
ii) the compound of formula III is then reacted with a strong base to produce a compound of the formula 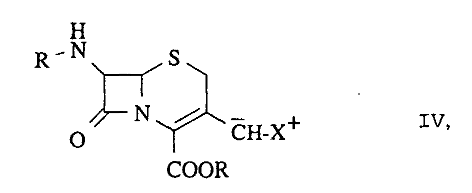 in which X⁺ is -P⁺(R₄)₃ or -[P(O)-(OR₄)₂] Y⁺, R₄ and R are as defined above and Y⁺ is a cation of the alkali series or the protonated form of a strong organic base; and
iii) the compound of formula IV is reacted with a compound of the formula 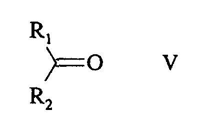 in which R₁ and R₂ are as defined above, to produce the compound of the formula I. The resulting process is simple, economical and may be carried out in a single reaction vessel. Also, it has the advantage that the silyl protection groups are removable by simple hydrolysis or alcoholysis.

The base used in step ii) is a strong organic base and guanidines (for example tetramethylguanidine), amidines (for example 1,8-diazabicyclo[5.4.0]undec-7-ene and 1,5-diazabicylo[4.3.0]non-5-ene), alkali salts of nitrogen-containing compounds (for example the Li or Na salts of 1,1,1,3,3,3-hexamethyldisilazane and Li-diisopropylamide), butyllithium, hydrides of alkali metals, and iminophosphoranes are given as suitable examples. It is also mentioned that the bases should be free of moisture and should not contain any parts that could be silylated, so as to maintain the degree of silylation of the product.

It has now been surprisingly found that the process described in EP 0503453 may be carried out using weaker bases. This is of particular advantage since the reaction may be carried out under milder conditions.

Therefore this invention provides a process, substantially as defined above, for the production of a compound of formula I which is improved in step ii) by the use of a weak base which is selected from the group consisting of:
i) compounds that have the formula 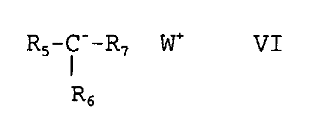 in which R₅ is hydrogen, alkyl or aryl; R₆ and R₇, which may be the same or different, are each an activated group of the formula -COOR₈, -CN, -SO₂R₈, -COR₈ or -CON(R₈)₂; or R₅ and R₆, which may be the same or different, are each aryl and R₇ is an activated group of the formula -COOR₈, -CN, -SO₂R₈, -COR₈ or -CON(R₈)₂; W⁺ is a cation (for example lithium, sodium, or calcium); and R₈ is alkyl, cycloalkyl or aryl; and
ii) salts of carboxylic acids of the formula R₁₀-COO⁻ W⁺ in which R₁₀ is an optionally branched alkyl group or an optionally substituted aryl group; and W⁺ is as defined above.

That a weaker base could be used in a Wittig reaction is indeed surprising. The use of the weaker base has the advantage that the possibility of opening the β-lactam ring is reduced and superfluous condensation of the base with the aldehyde or ketone is avoided or restricted.

Preferably the weak base is such that its conjugate acid has a silylatable function and the reaction step ii) is carried out in the presence of a silylating agent to cause silylation of the silylatable function. Surprisingly, the reaction proceeds without the silyl protecting group on the 7-amino group, which is a very potent silylating agent and which is easily removed, being removed by the base or conjugate acid. If the silyl protecting group were to be removed during the reaction, the amino group would be free to react with the aldehyde or ketone of formula V and this would cause the reaction to collapse.

Particularly preferred weak bases are lithium and sodium salts of malonic acid diethyl esters, acetoacetic acid esters, acetic acid, pivalic acid, or ethylhexanoic acids, or lithium salts of benzoic acids.

The silylating agent may be added to the reaction mixture prior to the addition of the weak base, which is a compound of formula VI or a salt of a carboxylic acid of formula R₁₀COO⁻W⁺ as defined above, or simultaneously with the weak base; in both cases to cause the silylation of silylatable function of the conjugate acid of the weak base. N,O-bis(trimethylsilyl)-acetamide and bissilylurea are particularly suitable as silylating agents and further examples are given in EP 0503453.

The reaction is carried out in a suitable solvent or solvent mixture which is inert under the reaction conditions, for example an inert ether (such as tetra-hydrofuran, diethyl ether, an ethylene glycol dialkyl ether or a tert.butylmethyl ether), an inert amide (such as dimethylformamide, dimethylacetamide or N-methylpyrrolidone), an urea (such as tetra-methylurea, 1,3-dimethyl-3,4,5,6-tetrahydro-2(1H)-pyrimidone, or 1,3,2-imidazolidinone) a nitrile (such as acetonitrile), or a halogenated hydrocarbon (such as dichloromethane).

Should a substituent of the aldehyde or the ketone of formula V contain a function which is easily silylated, this should be blocked temporarily with an appropriate silylation agent prior to the reaction. The amount of the compound of formula V may be stoichiometrical or in excess based on the amount of the compound of formula IV.

The reaction may be carried out over a wide temperature range, preferably at a temperature of between -70°C and +70°C.

The compounds of formula I may be isolated in a conventional manner. The silyl protecting groups may be removed by simple hydrolysis or alcoholysis. This may be done either by adding the desilylation agent to the reaction mixture, or by extracting the product into a separable aqueous phase, adding water (under alkaline or acidic conditions) and precipitating by adjusting the pH value to the isoelectric point, optionally adding an organic solvent.

The compounds of formula II are known and may be produced as described in EP 0503453.

The compounds of formula I are important starting materials for the production of valuable cephalosporin antibiotics. Cephalosporins which are vinyl-substituted in 3-position are either resorbed orally, or when administered parenterally, are known for their very broad, efficient spectrum of activity. The following compounds may be produced for example:

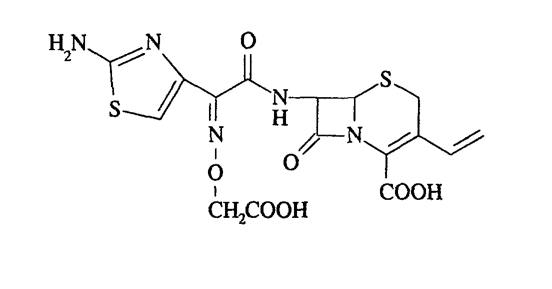

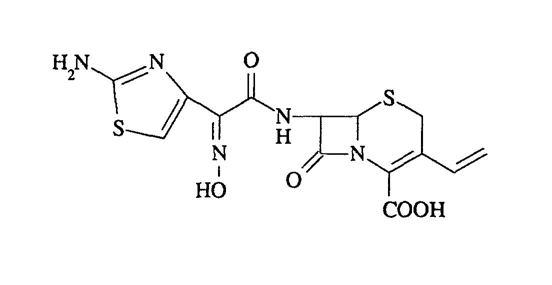

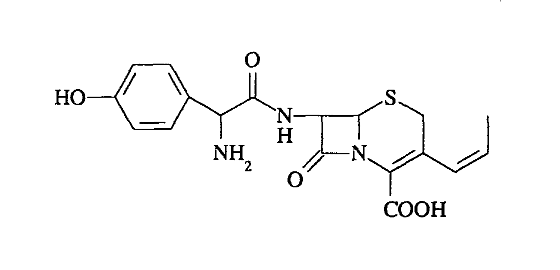

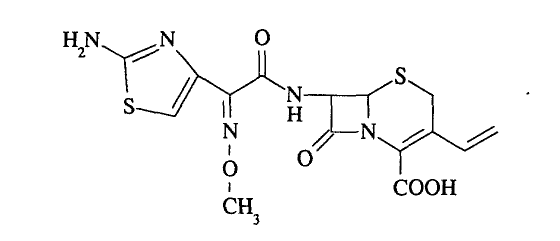

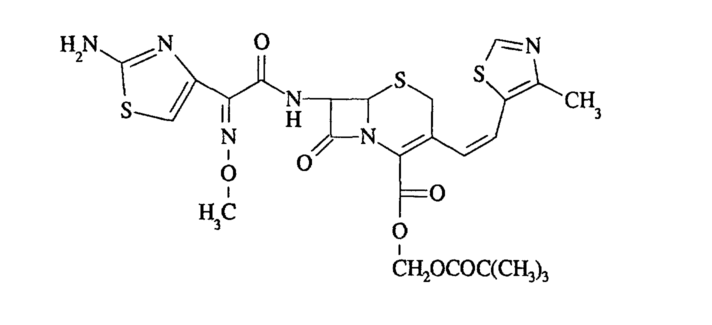

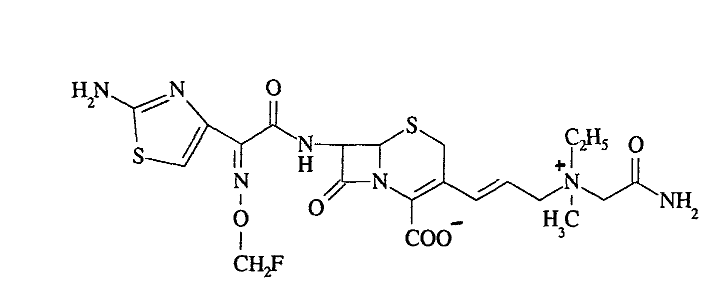

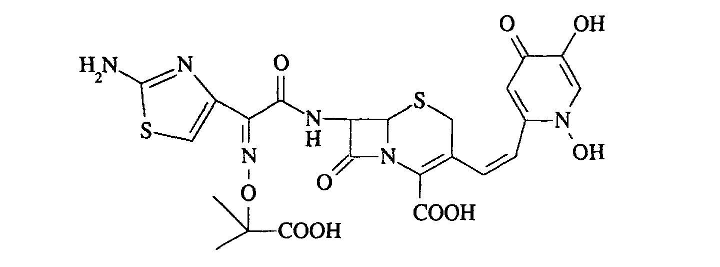

In the following examples, which illustrate the invention more fully, but in no way limit its scope, all temperatures are given in degrees celsius.

### Example 1: 7-Amino-3-(3-acetoxy-1-propenyl)-3-cephem-4-carboxylic acid

7.5 ml of N,O-bis(trimethylsilyl)acetamide is added to 25 ml of a dichloromethane solution containing 6 g of 7-trimethylsilylamino-3-triphenyl-phosphoniummethyl-3-cephem-4-carboxylic acid trimethylsilylester-iodide on ice. 25 ml of a N-Methylpyrrolidone solution, at 5 to 10°, containing 2.35 g sodium aceto-acetic acid ethyl ester, is added dropwise. The dark red solution is then cooled to 2° and 5.16 g acetoxy-acetaldehyde is added dropwise. The reaction mixture is then stirred for 2 hours at 10° and then added to a mixture of 100 ml acetic acid and 100 ml water. The pH of the aqueous phase is adjusted to 7 with ammonia and the organic phase is separated off. The pH is adjusted to 3.5 by adding 1:1 diluted concentrated HCl, whereupon the title compound precipitates. The suspension is stirred for 30 minutes at 5°, the title compound is filtered off, washed in acetone and dried.

### Example 2: 7-Amino-3-(prop-1-enyl)-3-cephem-4-carboxylic acid

5 ml of N,O-bis(trimethylsilyl)acetamide is added to 25 ml of a dichloromethane solution containing 6 g of 7-trimethylsilylamino-3-triphenyl-phosphoniummethyl-3-cephem-4-carboxylic acid trimethylsilylester-iodide on ice. 25 ml of a N-Methylpyrrolidone solution, at 5 to 10°, containing 2.35 g sodium malonic acid diethyl ester, is added dropwise. Thereafter the solution is cooled to -10° and 1.32 g acetaldehyde, dissolved in 10 ml dichloromethane, is added. After the addition of the acetaldehyde, the reaction mixture is stirred for 48 hours at 0°. Thereafter the process proceeds as described in example 1.

### Example 3: 7-Amino-3-(prop-1-enyl)-3-cephem-4-carboxylic acid

36.5 ml of N,O-bis(trimethylsilyl)acetamide is added to 500 ml of a dichloromethane solution containing 24 g of 7-trimethylsilylamino-3- triphenyl-phosphoniummethyl-3-cephem-4-carboxylic acid trimethylsilylester-iodide at a temperature of -10°. A suspension of 12.8 g lithium benzoate in 75 ml N-Methylpyrrolidone is added. 9 g of acetaldehyde is added and the reaction mixture is stirred for 2 days at 0°. Superfluous acetaldehyde and most of the dichloromethane are removed in a rotary evaporator. The residue is then stirred in 1500 ml of water and then filtered. The residue is dissolved in 200 ml aqueous ammonia and the aqueous phase is extracted twice using 100 ml dichloromethane. After removal of the organic phase, 1:1 diluted concentrated HCl is added to the aqueous phase to bring the pH to 3.5 whereupon the title compound precipitates. The suspension is stirred for 30 minutes at 5°, the title compound is filtered off, washed in acetone and dried.

### Example 4: 7-Amino-3-(3-acetoxy-1-propen-1-yl)-3-cephem-4-carboxylic acid

7.5 ml of N,O-bis(trimethylsilyl)acetamide is added to 25 ml of a dichloromethane solution containing 6 g of 7-trimethylsilylamino-3-triphenyl-phosphoniummethyl-3-cephem-4-carboxylic acid trimethylsilylester-iodide on ice. 15 ml of a N-methylpyrrolidone solution containing 2.5 g sodium-ethylhexanoate is added dropwise at 0 to 5°. 5 ml of acetoxy-acetaldehyde is added dropwise. The reaction mixture is stirred overnight at 0° and then processed as described in example 1.

### Example 5: 7-Amino-3-[2-(4-methyl-5-thiazolyl)vinyl]-3-cephem-4-carboxylic acid

25 g of a dichloromethane solution containing 10.2 g of 7-trimethylsilylamino-3-triphenyl-phosphoniummethyl- 3-cephem-4-carboxylic acid trimethylsilylester-iodide is cooled to a temperature of -10°. 4.7 ml of N,O-bis(trimethylsilyl)acetamide, 11 ml dimethylformamide and 1.1 g lithium acetate are added and the mixture stirred at 0 to 5° for 30 minutes. A solution of 2 g of 4-methyl-thiazol-5-carboxyaldehyde in 5 ml dichloromethane is then added dropwise. The mixture is then stirred for 10 hours at 30°, cooled to 10° and stirred for a further hour at 10°. The title compound is separated using a suction filter, washed with methanol and vacuum dried.

### Example 6: 7-Amino-3-(prop-1-enyl)-3-cephem-4-carboxylic acid

220.7 g of a dichloromethane solution containing 68.7 g of 7-trimethylsilylamino-3-triphenyl-phosphoniummethyl- 3-cephem-4-carboxylic acid trimethylsilylester-iodide is cooled to a temperature of -10°. 58.4 ml of N,O-bis(trimethylsilyl)acetamide and 81 ml N,N-dimethylacetamide is added while stirring. A solution of 14.05 g lithium pivalate is added and the mixture stirred for 30 minutes at -10°. 17.2 ml of acetaldehyde is added and the reaction mixture is stirred for 90 minutes at -10° and then overnight at 0°. Superfluous acetaldehyde and some of the dichloromethane are removed in a rotary evaporator under vacuum and at 20°. The residue is then stirred into 500 ml of ice-cold water and 100 ml dichloromethane. The pH value is adjusted to 8.5 with aqueous ammonia. The phases are then separated and the aqueous phase is washed with 100 ml dichloromethane and combined with 200 ml acetone. The pH is adjusted to 3.5 with 1:1 diluted concentrated hydrochloric acid at 30° to precipitate the title compound. The suspension is held in the ice bath for 2 hours whilst stirring, and the title compound is isolated using a suction filter. The title compound is washed with a mixture of 100 ml of water and 50 ml acetone and then again with 50 ml of acetone. The title compound is then dried in a vacuum drying chamber at 40°.

## Claims

1. A process for the production of a 3-vinylcephalosporin compound of formula 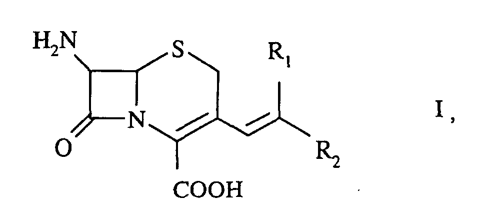 wherein R₁ and R₂ may be the same or different and denote hydrogen or
(i) an optionally branched alkyl, alkenyl or alkinyl group;
(ii) a totally or partially saturated cycloalkyl radical; or
(iii) an optionally substituted aryl radical, aralkyl radical or heterocycle,
wherein the organic radicals specified in (i), (ii) and (iii) contain up to 10 carbon atoms and
wherein the cycloalkyl radical, aryl radical, aralkyl radical or heterocycle are optionally substituted by a halogen, nitrogen, sulphur, alkoxy, aryloxy, a carbalkoxy or carboxamido group;
carried out in a solvent or solvent mixture which is inert under the reaction conditions,
which comprises the steps of
i) reacting a compound of formula 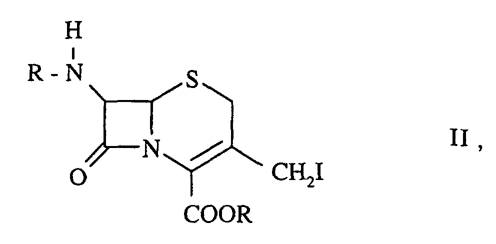 wherein R is a silyl protecting group,
with a compound of the formula P(R₄)₃ or P(OR₄)₃,
wherein R₄ is an alkyl group of 1 to 4 carbon atoms or an aryl group;
to produce a compound of formula 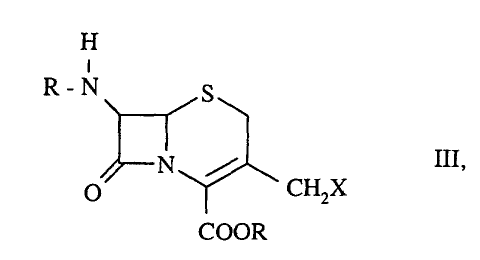 wherein
X is -P(R₄)₃I or -P(O)(OR₄)₂, wherein R₄ is as defined above; and
R is as defined above; and
ii) reacting a compound of formula III with a base to produce a compound of formula 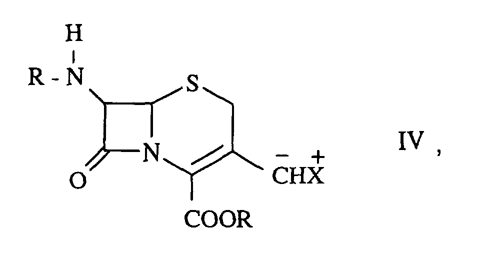 wherein
R is as defined above,
X⁺ is -P⁺(R₄)₃ or -[P(O)(OR₄)₂Y]⁺,
wherein R₄ is as defined above, and
Y is a cation of the alkali series or the protonated form of the base; and
wherein the reaction is performed in the presence of a silylating agent; and
iii) reacting a compound of formula IV with a compound of formula 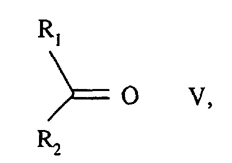
wherein R₁ and R₂ are as defined above, to produce a compound of formula I;
**characterised in that** the base reacted with a compound of formula III in step ii) is
i) a compound of formula 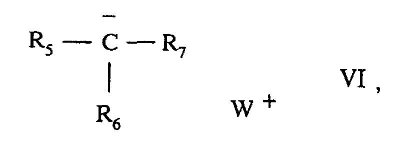 wherein
R₅ is hydrogen, alkyl or aryl;
R₆ and R₇, which may be the same or different, are each an activated group of formula -COOR₈, -CN, -SO₂R₈, -COR₈ or -CON(R₈)₂; or,
R₅ and R₆, which may be the same or different, are each aryl and R₇ is an activated group of formula -COOR₈, -CN, -SO₂R₈, -COR₈ or -CON(R₈)₂;
wherein R₈ is alkyl, cycloalkyl or aryl; and
W⁺ is a cation; or,
ii) a salt of a carboxylic acid of formula R₁₀-COO⁻ W⁺ wherein
R₁₀ is an optionally branched alkyl group or an optionally substituted aryl group; and W⁺ is as defined above.

2. A process according to claim 1, wherein the compound of formula VI or the carboxylic acid salt of formula R₁₀-COO⁻ W⁺ is a lithium or sodium salt of malonic acid diethyl ester, acetoacetic acid ester, acetic acid, pivalic acid, or ethylhexanoic acid, or is a lithium salt of benzoic acid.

3. A process according to any one of claims 1 or 2, wherein the silylating agent is added to the reaction mixture prior to the addition of the base, which is a compound of formula VI or a carboxylic acid salt of formula R₁₀-COO⁻ W⁺ as defined in claim 1.

4. A process according to any one of claims 1 or 2, wherein the silylating agent is added to the reaction mixture at the same time as the base, which is a compound of formula VI or a carboxylic acid salt of formula R₁₀-COO⁻ W⁺ as defined in claim 1.

5. A process according to any of claims 1 to 4, wherein the silylating agent is N,O-bis(trimethylsilyl)acetamide or bissilylurea.

6. Use of a base of formula VI or of formula R₁₀-COO⁻ W⁺ as defined in claim 1 to produce an ylide of formula IV, as defined in claim 1, in a reaction according to claim 1.

7. Use according to claim 6 in a process for the production of a 3-vinylcephalosporin compound of formula I, as defined in claim 1.

## Patentansprüche

1. Verfahren zur Herstellung einer 3-Vinylcephalosporinverbindung der Formel 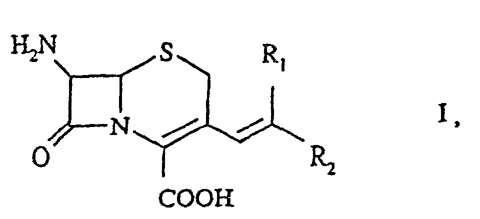 worin R₁ und R₂ gleich oder verschieden sein können und stehen für Wasserstoff oder
(i) eine wahlweise verzweigte Alkyl-, Alkenyl- oder Alkinylgruppe,
(ii) einen vollkommen oder teilweise gesättigten Cycloalkylrest, oder
(iii) einen wahlweise substituierten Arylrest, Aralkylrest oder Heterocyclus,
worin die organischen Reste, die in (i), (ii) und (iii) spezifiziert sind, bis zu 10 Kohlenstoffatome enthalten und worin der Cycloalkylrest, der Arylrest, der Aralkylrest oder der Heterocyclus wahlweise substituiert sind durch Halogen, Stickstoff, Schwefel, Alkoxy, Aryloxy, eine Carbalkoxy- oder Carboxamidogruppe,
das in einem Lösemittel oder Lösemittelgemisch ausgeführt wird, das unter den Reaktionsbedingungen inert ist,
das die folgenden Schritte umfaßt
i) Umsetzung einer Verbindung der Formel 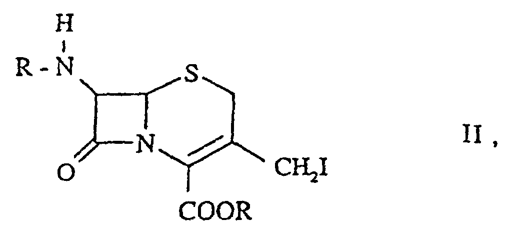 worin R für eine Silylschutzgruppe steht,
mit einer Verbindung der Formel P(R₄)₃ oder P(OR₄)₃,
worin R₄ für eine Alkylgruppe mit 1 bis 4 Kohlenstoffatomen oder eine Arylgruppe steht
unter Bildung einer Verbindung der Formel 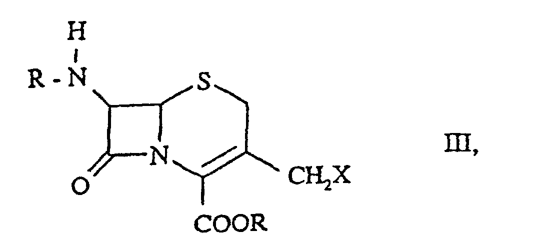 worin
X für -P(R₄)₃I oder -P(O)(OR₄)₂ steht, worin R₄ wie oben definiert ist, und
R wie oben definiert ist, und
ii) Umsetzung einer Verbindung der Formel III mit einer Base unter Bildung einer Verbindung der Formel 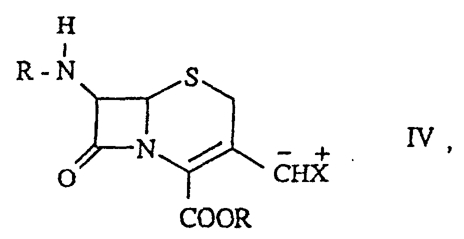 worin
R wie oben definiert ist,
X⁺ für -P⁺(R₄)₃ oder -[P(O)(OR₄)₂Y]⁺ steht,
worin R₄ wie oben definiert ist, und
Y für ein Kation der Alkalireihe oder die protonierte Form einer Base steht und worin die Reaktion in Gegenwart eines Silylierungsmittels ausgeführt wird, und
iii) Umsetzung einer Verbindung der Formel IV mit einer Verbindung der Formel 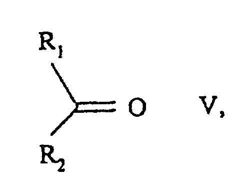 worin R₁ und R₂ wie oben definiert sind, unter Bildung einer Verbindung der Formel I,
**dadurch gekennzeichnet, daß** die Base, die mit einer Verbindung der Formel III in Schritt ii) umgesetzt wird, folgende ist
i) eine Verbindung der Formel 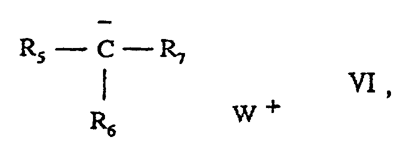 worin
R₅ für Wasserstoff, Alkyl oder Aryl steht,
R₆ und R₇, die gleich oder verschieden sein können, jeweils für eine aktivierte Gruppe der Formel -COOR₈, -CN, -SO₂R₈, -COR₈ oder -CON(R₈)₂ stehen, oder
R₅ und R₆, die gleich oder verschieden sind, jeweils für Aryl stehen und R₇ für eine aktivierte Gruppe der Formel -COOR₈, -CN, -SO₂R₈, -COR₈ oder -CON(R₈)₂ steht,
worin R₈ für Alkyl Cycloalkyl oder Aryl steht, und
W⁺ für ein Kation steht, oder
ii) ein Salz einer Carbonsäure der Formel R₁₀-COO⁻W⁺, worin
R₁₀ für eine wahlweise verzweigte Alkylgruppe oder eine wahlweise substituierte Arylgruppe steht und W⁺ wie oben definiert ist.

2. Verfahren nach Anspruch 1, worin die Verbindung der Formel VI oder das Carbonsäuresalz der Formel R₁₀-COO⁻ W⁺ ein Lithium- oder Natriumsalz von Malonsäurediethylester, Acetoessigsäureester, Essigsäure, Pivalinsäure oder Ethylhexansäure ist oder ein Lithiumsalz der Benzoesäure ist.

3. Verfahren nach einem der Ansprüche 1 oder 2, worin das Silylierungsmittel vor der Zugabe der Base zum Reaktionsgemisch gegeben wird, die eine Verbindung der Formel VI oder ein Carbonsäuresalz der Formel R₁₀-COO⁻W⁺ ist, wie dies in Anspruch 1 definiert ist.

4. Verfahren nach einem der Ansprüche 1 oder 2, worin das Silylierungsmittel zur selben Zeit wie die Base zum Reaktionsgemisch gegeben wird, die eine Verbindung der Formel VI oder ein Carbonsäuresalz der Formel R₁₀-COO⁻W⁺ ist, wie dies in Anspruch 1 definiert ist.

5. Verfahren nach einem der Ansprüche 1 bis 4, worin das Silylierungsmittel N,O-Bis(trimethylsilyl)acetamid oder Bissilylharnstoff ist.

6. Verwendung einer Base der Formel VI oder der Formel R₁₀-COO⁻W⁺ nach Anspruch 1 zur Herstellung eines Ylids der Formel IV, wie dies in Anspruch 1 definiert ist, in einer Reaktion nach Anspruch 1.

7. Verwendung nach Anspruch 6 in einem Verfahren zur Herstellung einer 3-Vinylcephalosporinverbindung der Formel I, wie sie in Anspruch 1 definiert ist.

## Revendications

1. Procédé de préparation d'un composé de 3-vinylcéphalosporine de formule : 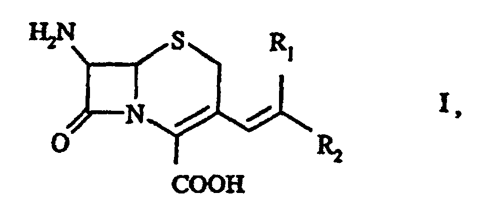 dans laquelle R₁ et R₂ peuvent être identiques ou différents et désignent l'hydrogène ou
(i) un groupe alkyle, alcényle ou alcynyle éventuellement ramifié ;
(ii) un radical cycloalkyle totalement ou partiellement saturé ; ou
(iii) un radical aryle, aralkyle ou un hétérocycle éventuellement substitué,
où les radicaux organiques indiqués en (i), (ii) ou (iii) contiennent jusqu'à 10 atomes de carbone, et où le radical cycloalkyle, le radical aryle, le radical aralkyle ou l'hétérocycle sont facultativement substitués par un halogène, l'azote, le soufre, un groupe alcoxy, aryloxy, carbalcoxy ou carboxamido;
conduit dans un solvant ou mélange de solvants qui est inerte dans les conditions réactionnelles,
qui comprend les étapes consistant à :
i) faire réagir un composé de formule : 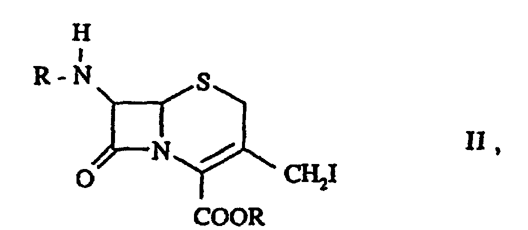 dans laquelle R est un groupe protecteur silyle,
avec un composé de formule P(R₄)₃ ou P(OR₄)₃
dans laquelle R₄ est un groupe alkyle ayant 1 à 4 atomes de carbone ou un groupe aryle ;
pour donner un composé de formule : 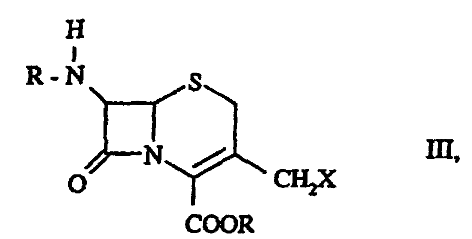 dans laquelle X représente -P(R₄)₃I ou -P(O)-(OR₄)₂, où R₄ est tel que défini ci-dessus ; et R est tel que défini ci-dessus ; et
ii) faire réagir un composé de formule III avec une base pour donner un composé de formule : 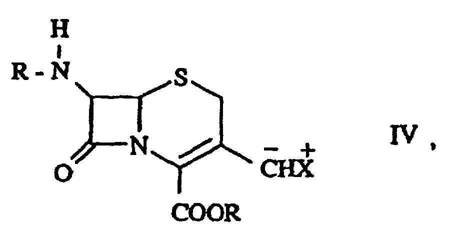 dans laquelle
R est tel que défini ci-dessus,
X⁺ représente -P⁺(R₄)₃ ou [P(O)-(OR₄)₂]Y⁺,
où R₄ est tel que défini ci-dessus, et
Y est un cation des séries alcalines ou de la forme protonée de la base ; et
dans laquelle la réaction est conduite en présence d'un agent de silylation ; et
iii) faire réagir un composé de formule IV avec le composé de formule : 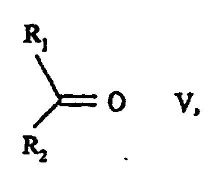 dans laquelle R₁ et R₂ sont tels que définis ci-dessus, pour donner un composé de formule I;
**caractérisé en ce que** la base mise à réagir avec un composé de formule III dans l'étape ii) est :
i) un composé de formule : 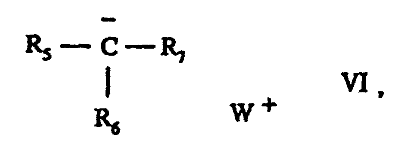 dans laquelle :
R₅ représente l'hydrogène, un groupe alkyle ou aryle;
R₆ et R₇, qui peuvent être identiques ou différents, représentent chacun un groupe activé de formule -COOR₈, -CN, -SO₂R₈, -COR₈ ou -CON(R₈)₂ ; ou bien
R₅ et R₆, qui peuvent être identiques ou différents, représentent chacun un groupe aryle et R₇ est un groupe activé de formule -COOR₈, -CN, -SO₂R₈, -COR₈ ou -CON(R₈)₂;
où R₈ représente un groupe alkyle, cycloalkyle ou aryle ; et
W⁺ est un cation ; ou bien
ii) un sel d'un acide carboxylique de formule R₁₀-COO⁻W⁺, où
R₁₀ est un groupe alkyle facultativement ramifié ou un groupe aryle éventuellement substitué ; et W⁺ est tel que défini ci-dessus.

2. Procédé suivant la revendication 1, dans lequel le composé de formule VI ou le sel d'acide carboxylique de formule R₁₀-COO⁻W⁺ est un sel de lithium ou de sodium du diéthylester de l'acide malonique, de l'ester d'acide acétoacétique, de l'acide acétique, de l'acide pivalique, ou de l'acide éthylhexanoïque, ou est un sel de lithium de l'acide benzoïque.

3. Procédé suivant l'une quelconque des revendications 1 ou 2, dans lequel l'agent de silylation est ajouté au mélange réactionnel avant l'addition de la base, qui est un composé de formule VI ou un sel d'acide carboxylique de formule R₁₀-COO⁻W⁺ tel que défini dans la revendication 1.

4. Procédé suivant l'une quelconque des revendications 1 ou 2, dans lequel l'agent de silylation est ajouté au mélange réactionnel en même temps que la base, qui est un composé de formule VI, ou un sel d'acide carboxylique de formule R₁₀-COO⁻W⁺ tel que défini dans la revendication 1.

5. Procédé suivant l'une quelconque des revendications 1 à 4, dans lequel l'agent de silylation est le N,O-bis(triméthylsilyl)acétamide ou la bis-silylurée.

6. Utilisation d'une base de formule VI, ou de formule R₁₀-COO⁻W⁺ telle que définie dans la revendication 1, pour préparer un ylure de formule IV, de la manière définie dans la revendication 1, dans une réaction suivant la revendication 1.

7. Utilisation suivant la revendication 6 dans un procédé de préparation d'un composé de 3-vinylcéphalosporine de formule I, tel que défini dans la revendication 1.
